# EUROPEAN PATENT APPLICATION

(11) **EP 2 939 664 A1**
(43) Date of publication of application: **04.11.2015**
(21) Application number: 13868552.4
(22) Date of filing: 24.12.2013
(51) Int. Cl.: A61K 9/19, A61K 47/30

(54) **FREEZE-DRIED EXCIPIENT AND PREPARATION METHOD THEREOF**

(30) Priority: 26.12.2012 CN 201210572660
(71) Applicant: Li, Hewei, Beijing 101312 (CN)
(72) Inventor: Li, Hewei, Beijing 101312 (CN)
(74) Representative: Kugler, Jörg
(86) International application number: PCT/CN2013/090291
(87) International publication number: WO 2014/101743

(57) **Abstract**

A freeze-dried excipient and a preparation method thereof. The freeze-dried excipient only comprises an active ingredient and a binder, and does not comprise a support agent. The weight ratio of the active ingredient to the binder is 1:100 to 100:1. The binder is adhesives, cellulose ethers, modified starches, PVP, carbomer, PVA, hyaluronic acids, albumin, chitosan, dextran, agar, polyamino acid, glycan or a combination thereof.

## Description

The present application claims the priority of Chinese patent application No. 201210572660.8, filed on 26, Dec., 2012, to the Patent Office of the People's Republic of China, and titled "Freeze-dried excipient and preparation method thereof", which is hereby incorporated by reference in its entirety.

### FIELD OF THE INVENTION

The present invention relates to the formula of a freeze-dried forming preparation and the preparation method thereof, and particularly to the formula of the freeze-dried forming preparation containing only a binder and an active ingredient but no matrix forming agent (such as amino acids, sugars, sugar alcohols and inorganic salts etc.) and the preparation method thereof.

### BACKGROUND OF THE INVENTION

The technique for shaping by freeze-drying refers to a shaping technique comprising: injecting a flowable liquid, semisolid or solid containing an active ingredient into which a matrix forming agent and a binder are added, or a flowable liquid, semisolid or solid containing a binder and a matrix forming agent in itself into a mould and then shaping by freeze-drying. The formulation prepared by the shaping technique by freeze-drying is referred as a freeze-dried forming preparation.

Since this type of formulation is prepared using a freeze-drying process, the degradation of the thermosensitive ingredient can be avoided. Besides, the formulation may have a fast disintegration and dissolution rate due to a great amount of micropores and channels produced by water sublimation, so that it is suitable for application in a plurality of fields, such as oral disintegrating tablets, immediate release tablets, chewable tablets and special cosmetics.

Conventional freeze-dried forming preparation is composed of two parts, a matrix and an active ingredient, in which the matrix comprises a matrix forming agent and a binder. The matrix forming agent is mostly selected from the group comprising sugars, sugar alcohols, amino acids having 2 to 12 carbon atoms and inorganic salts (such as sodium phosphate, and aluminum silicate *etc.)* and so on (See Chinese patent CN200580013010.8).

When sugars, sugar alcohols, or inorganic salts (such as sodium phosphate, and aluminum silicate *etc.*) are used as the matrix forming agent, collapse or complete collapse of the skeleton structure ingredient may occur, since the moisture in the air can be easily absorbed by the sugars, sugar alcohols, or inorganic salts (such as sodium phosphate, and aluminum silicate *etc.*) during manufacture. In addition, bad feelings due to heavy hygroscopicity, such as sticky to hand, may also happen when the package is opened by the user. Besides, it also has many disadvantages, such as prolonged disintegration *etc.* Although the hygroscopicity can be reduced when amino acids are used as the matrix forming agent, it can not be solved fundamentally. In addition, the cost of the production process is still a problem.

Accordingly, there is still a demand for a freeze-dried forming preparation without a matrix forming agent. However, due to the technical limitation and traditional concept, there is no report on the freeze-dried forming preparation prepared using an active ingredient and a binder only. Specific description for this respect can be found in patent 200580013010 and patent 200410038822.

### SUMMARY OF THE INVENTION

The technical problem intended to be solved in the present invention is to provide a simplified formulation containing only a binder and an active ingredient, but no matrix forming agent in the system. The matrix forming agent includes, but not limited to, sugars (such as maltose, and trehalose *etc*.), sugar alcohols (such as mannitol, and sorbitol), amino acids having 2 to 12 carbon atoms (such as glycine, alanine, and glutamic acid *etc*.), inorganic salts (such as sodium phosphate, and aluminium silicate *etc.*) and so on.

After a great amount of experiments, the inventors have found that a qualified freeze-dried forming preparation can be obtained using a binder and an active ingredient only, but no matrix forming agent system by controlling the ratio between the binder and the active ingredient. On this basis, other adjuvants, such as antioxidant, flavoring agent and essence, transdermal absorption enhancer, and pH adjusting agent *etc,* can be added. By avoiding the use of a matrix forming agent, favorable effects, such as reduced risk for moisture absorption, simplified process, reduced cost, and enhanced unit drug loading, can be achieved.

The present invention will be described in detail below.

The freeze-dried forming preparation provided in the present invention comprises only two parts: a binder and an active ingredient, wherein the ratio by weight between the binder and the active ingredient is in the range from 1:100 to 100:1. It has been demonstrated by the experiments that qualified freeze-dried forming preparation can not be prepared beyond this range. The ratio by weight between the binder and the active ingredient can be further preferably in the range from 1:90 to 90:1, 1:80 to 80:1, 1:70 to 70:1, 1:60 to 60:1, 1:50 to 50:1, 1:40 to 40:1, and 1:30 to 30:1, and more preferably, 1:20 to 20:1, 1:10 to 10:1, 1:9 to 9:1, 1:8 to 8:1, and 1:7 to 7:1, and most preferably, 1:6 to 6:1, and 1:5 to 5:1. The freeze-dried forming preparation provided in the present invention can further comprise other adjuvants, for example, antioxidant, flavoring agent and essence, transdermal absorption enhancer, and pH adjusting agent *etc.* The other adjuvants can be present in the amount of 0.1-5%, and preferably, 0.1-3%, based on the total content of the obtained freeze-dried forming preparation.

The active ingredient can be water-soluble or water-insoluble. The active ingredient can be selected from the combination of one or more of chemical medicine ingredients, traditional Chinese medicine ingredients, natural extracts, bioactive ingredients and beneficial ingredients for skin care.

There is no special limitation on the active ingredient involved in the present invention, and it can be selected from, but not limited to, one or more of the ingredients below.

### Chemical medicines (pharmaceutically active ingredients):

Antipyretic, analgesics and anti-inflammatory drugs, for example, aspirin, diflunisal, salsalate, paracetamol, indometacin, ibuprofen, naproxen, ketoprofen, pirprofen, suprofen, flurbiprofen, piroxicam, meloxicam, nimesulide, and benzbromarone *etc.*;
Central stimulants, for example, pemoline, adrafinil, and piracetam *etc.*;
Drugs for migraine, for example, sumatriptan succinate;
Analgesic drugs, for example, rotundine, buprenorphine, pentazocine, and naloxone *etc.*;
Drugs for Parkinson's disease and Alzheimer's disease, for example, L-dopa, compound carbidopa, compound benserazide, amantadine hydrochloride, piribedil, profenamine, donepezil, and huperzine A *etc.*;
Antipsychataxia drugs, for example, chlorpromazine, phenergan, pethidine, thioridazine, chlorprothixene, clozapine, sulpiride, tiapride, penfluridol, and risperidone *etc.;*
Antiepileptic drugs and anticonvulsants, for example, phenytoin, carbamazepine, primidone, gabapentin, lamotrigine, sodium valproate, and clonazepam *etc.*
Sedative-hypnotics, for example, diazepam, nitrazepam, oxazepam, lorazepam, and phenobarbital *etc.*;
Cholinesterase inhibitors, for example, scopolamine *etc.*;
Antiarrhythic drugs, for example, propyl pyridine, tocainide, mexiletine, ethmozine, phenytoin, propafenone, and amiodarone *etc.;*
Antianginal and antiatherosclerotic drugs, for example, propranolol, nifedipine, gemfibrozil, bezafibrate, lovastatin, simvastatin, and pravastatin *etc.;*
Antihypertensive drugs, for example, enalapril, captopril, hydrochlorothiazide, and amlodipine *etc.;*
Adrenergic receptor blockers, for example, acebutolol, and alprenolol *etc.;*
Corticosteroids, for example, betamethasone, and cortisone acetate *etc.*;
Antidiabetic drugs, for example, repaglinide *etc.*;
Antithyroid drugs, for example, propylthiouracil, carbimazole, and methimazol *etc.*;
Antihistamines, for example, cetirizine hydrochloride, and loratadine *etc.;*
Autacoids, for example, dinoprostone, alprostadil, and betahistine *etc.*;
Drugs for digestive system, for example, scopolamine butylbromide, and granisetron hydrochloride *etc.*;
Drugs for blood system, for example, EPO, and cobamamide *etc.*;
Drugs for urinary system, for example, azosemide, and furosemide *etc.*;
Drugs for reproductive system, for example, estrogen, and nandrolone phenylpropionate *etc.*;
Antiparasitic drugs, for example, albendazole, and cambendazole *etc.;*
Antineoplastic drugs, for example, aminoglutethimide, and amsacrine *etc.;*
Antimicrobial drugs, for example, ampicillin, and sulbenicillin sodium *etc.*;
Antibiotics, for example, amoxicillin, cefalexin, cefprozil, cefuroxime axetil, roxithromycin, erythromycin ethylsuccinate, and josamycin *etc.*

### Traditional Chinese medicine ingredients:

Active components of Chinese traditional medicines, such as, breviscapine, arteannuin, huperzine A, and corydalis B *etc.;*
Extracts of a single traditional Chinese medicine or extracts of compound traditional Chinese medicine, such as, tanshinone extract, total phenolic acid extract of *radix salviae miltiorrhizae*, extract of compound Danshen dripping pills, extract of compound Niuhuang Shangqing pills, total saponin of stem and leaf of ginseng, extract of *Rhizoma Menispermi,* total saponin of ginseng, total saponin of American ginseng, breviscapine, Glabrous Sarcandra herb extract, total saponin of notoginseng, oriental wormwood extract, rhubarb extract, andrographolide, hawthorn leaf extract, total glycosides of centella and ginkgo leaf extract *etc.*
**Natural plant extracts,** such as, aloe extract, Chinese yam extract, cowberry extract, balsam pear extract, *Echinacea purpurea* extract, feverfew extract, mangosteen extract, pine needle and pine bark extract, acai berry extract, mulberry extract, elderberry extract, cranberry extract, astaxanthin, lycopene, green tea extract, grape pip and grape skin extract, glabridin, penoniflorin, licoflavone, extract of root-bark of tree peony *etc.*
**Bioactive ingredients:** EGF, bFGF, aFGF, KGF, IGF, NGF, TGF, and HGH *etc.*
**Benifical ingredients for skin care:** vitamin A, vitamin B1, vitamin B2, vitamin B3, vitamin B6, vitamin B12, vitamin C, vitamin D, vitamin E, vitamin K, Coenzymes, proteases, metallothioneins, pearl and its hydrolysate, milk and its extract, pollen and its extract, royal jelly, and propolis *etc.*

The binder is a water soluble high molecular material that is either edible or pharmaceutically acceptable, which can be polysaccharides, polypeptides, and proteins, and can be artificial polymacromolecules, or modified natural high molecular materials or the mixture thereof. Common binders include, but not limited to, gums (collagen, gelatin, hydrolyzed gelatin, acacia, xanthan gum, carrageenin, pectin, konjac glucomannan, carrageenin, locust bean gum, wood gum, locust bean gum *etc*.), cellulose ethers (carboxymethyl cellulose, carboxyethyl cellulose, hydroxyethyl methylcellulose, hydroxypropyl methylcellulose *etc*.), modified starches (pullulan, hydroxypropyl starch *etc.,* see R. P. Scherer US4305502A), PVP, PVA, hyaluronic acids, albumin, chitosan, dextran, agar, polyamino acids, polysaccharides and the combination thereof *etc.*; which are characterized in that the gum-derived binders are selected from gelatin, hydrolyzed gelatin, acacia, xanthan gum, carrageenin, pectin, konjac glucomannan, carrageenin, locust bean gum, wood gum, locust bean gum; the cellulose ether-derived binders are selected from carboxymethyl cellulose, carboxyethyl cellulose, hydroxyethyl methylcellulose, hydroxypropyl methylcellulose *etc.*; the modified starch-derived binders are selected from pullulan, hydroxypropyl starch, hydroxylpropylmethyl starch, pregelatinized starch, amylose, carboxymethyl starch, hydroxyethyl starch, hydroxypropyl starch *etc.*; the polyamino acids are selected from polyglutamic acid, polyalanine, polylysine *etc.*; the polysaccharides are selected from fucoidin, and inulin *etc.*

The antioxidants include, but not limited to, one or more of vitamin C and its derivatives, cyanidin, resveratrol and polyatomic phenol compounds of plant origin;
The flavoring agents and essences include, but not limited to, one or more of mint taste, chocolate taste, fruit taste, vanilla taste, coffee taste, tea taste, maize taste, lemon taste and milk taste *etc.*

The transdermal absorption enhancers include, but not limited to, one or more of lecithin, saponin, sodium lauryl alcohol acid, azone, tween and span;
The pH adjusting agents include, but not limited to, one or more of citric acid, tartaric acid, carbonate, sodium carbonate and phosphate.

Another aspect of the present invention relates to the preparation method of the freeze-dried forming preparation mentioned above, which comprises:
I. The preparation method of the freeze-dried forming preparation without any other adjuvant:
   (a) injection molding is performed to the solution or suspensoid composed of an active ingredient, water and a binder; or injection molding is performed to a solid active ingredient and/or a binder, followed by adding water to form a suspension;
   (b) the solution or suspensoid obtained from step (a) is degassed in a quantitative mould;
   (c) the degassed suspensoid from step (b) is frozen or the suspension from step (a) is frozen directly without degassing at low temperature;
   (d) the formulation from step (c) is freeze-dried in a quantitative mould to obtain a freeze-dried forming preparation.
II. The preparation method of the freeze-dried forming preparation containing other adjuvant:
   (a) injection molding is performed to the suspensoid composed of an active ingredient, water, a binder and other adjuvant; or injection molding is performed to a solid active ingredient and/or a binder and/or other adjuvant, followed by adding water to form a suspension;
   (b) the solution or suspensoid obtained from step (a) is degassed in a quantitative mould;
   (c) the degassed solution or suspensoid from step (b) is frozen or the solution or suspension from step (a) is frozen directly without degassing at low temperature;
   (d) the formulation from step (c) is freeze-dried in a quantitative mould to remove the solvent and obtain a freeze-dried forming preparation.

In both methods above, the ratio by weight between the active ingredient together with the binder and water is from 100:1 to 1:100, and it can be further preferably in the range from 1:90 to 90:1, 1:80 to 80:1, 1:70 to 70:1, 1:60 to 60:1, 1:50 to 50:1, 1:40 to 40:1, and 1:30 to 30:1, and more preferably, 1:20 to 20:1, 1:10 to 10:1, 1:9 to 9:1, 1:8 to 8:1, and 1:7 to 7:1, and most preferably, 1:6 to 6:1, and 1:5 to 5:1.

The injection molding of liquid can be carried out by precisely quantitative glass pipette, pipette, or electronic pipette, or it also can be performed by a plunger pump, a gear pump or a peristaltic pump *etc.* The prepared solution, suspensoid or suspension can be injected into a quantitative mould. The injection molding of solid can be carried out by a precise solid measuring implement or a shaking capillary powder flow controller;

The method for degassing can be selected from centrifugal degassing, vacuum degassing, and ultrasonic degassing *etc.;*

Freezing can be performed by spraying liquid nitrogen, or liquid or solid carbon dioxide, or in a manner of circulating jacket cooling, refrigeration by turbo-expander or cascade system refrigeration, at temperature from -20°C to -196°C to allow the solution, suspensoid or suspension frozen into solid rapidly.

The vacuum for freeze drying is from 0.01 to 20 millibar, and the temperature is in the range from -70°C to 50°C.

### DETAILED EMBODIMENTS

Freeze-dried forming preparation and preparation method thereof are disclosed by the present invention, which can be implemented by properly modifying the processing parameters by those skilled in the art with reference to the content herein. Particularly, it should be noted that all similar replacements and modifications are apparent to those skilled in the art, all of which are regarded to be included in the present invention. The method of the present invention and the applications thereof have been described by preferred Examples, and it is apparent that modification, or proper change and the combination thereof can be made to the method and applications described herein by those skilled in the art, without departing from the content, spirit and scope of the invention, in order to achieve and apply the techniques disclosed in the present invention.

The present invention will be further described by the Examples below, and it is not intended that the present invention is limited to these Examples.

In the Examples and Comparative Examples below, the evaluation methods for the freeze-dried forming preparation are described as follows:

### 1) Disintegration time:

The formulation is placed into a tube containing 2 ml water (37°C), and the time for complete disintegration and passing through a 20-mesh sieve is recorded;

### 2) Friability:

The formulation is allowed to fall from a position of 1 meter high down to a stainless steel plate in a manner of free falling body. After 100 pellets of the formulation have been fallen, the total weight of the powder left from the pellet is measured. The formulation is regarded as qualification when the total weight of the powder left from the pellet is less than 3% of the total weight of the formulation.

### 3) Appearance of the formulation:

The prepared freeze-dried forming preparation is taken out of the bottom of the aluminium nest by a finger with an upward force. The appearance, smoothness, and defects such as pit, or crackle are inspected visually.

### Example 1:

Vitamin C: collagen = 100:1. 100 mg vitamin C was precisely injected into a 0.3 ml mould. After dissolved in 0.2 ml water, 1 mg collagen was injected into the mould containing 100 mg vitamin C. The mixture was stirred to disperse the water in the powder, and was quickly frozen to -100°C. The mixture was freeze-dried to obtain a solid beverage.

### Example 2:

Pearl powder: hyaluronic acid = 1:100. hyaluronic acid 100mg, wherein 90 mg hyaluronic acid was mixed with 1 mg pearl powder, and the mixture was subsequently precisely injected into a 0.5 ml mould. After dissolved in 0.3 ml water, another 10 mg hyaluronic acid was injected into the mould containing 90 mg hyaluronic acid and 1 mg pearl powder. The mixture was stirred to disperse the water in the powder, and was quickly frozen to -196°C. The mixture was freeze-dried to obtain a solid skin-care essence.

### Example 3:

Cowberry extract: pullulan = 5:1. 60 mg cowberry extract and 12 mg pullulan were added into 0.4 ml water to prepare a solution, which was subsequently injected into a 0.4 ml mould, and freeze-dried to obtain a solid beverage.

### Example 4:

Total saponin of notoginseng: PVP = 10:1. 500 mg total saponin of notoginseng and 50mg PVP, wherein 100 mg total saponin of notoginseng and 10 mg PVP were injected into a 0.5 ml mould in a form of powder, which were subsequently dispersed by a solution composed of 40 mg PVP and 0.5 ml water. The mixture was freeze-dried to obtain buccal tablets.

### Example 5:

Total flavone of gingko: PVA = 1:1 : 20 mg total flavone of gingko and 20 mg PVA were added to 0.3 ml water to prepare a solution, which was then injected into a 0.3 ml mould and freeze-dried to obtain buccal tablets.

### Example 6:

Resveratrol: polylysine = 5:1. 100 mg resveratrol and 20 mg polylysine were prepared into a suspensoid, which was then injected into a 0.4 ml mould and freeze-dried to obtain buccal tablets.

### Example 7:

Loratadine: dextran = 1:10. 10 mg loratadine and 100 mg dextran, wherein 10 mg loratadine and 50 mg dextran were dispersed into a 0.2 ml mould as powder. A solution was prepared using another 50 mg dextran and 0.2 ml water, and injected into the 0.2 ml mould. The solution was mixed with the powder in the mould, stirred and freeze-dried to obtain buccal tablets.

### Example 8:

Paracetamol: PVA = 25:1. 500 mg paracetamol and 20 mg PVA were injected into a 1 ml mould. The mixture was divided into 2 parts. 500 mg paracetamol powder was injected into a 0.6 ml mould. 20 mg PVA was dissolved in 0.4 ml water, and then injected into 500 mg powder. After stirred and freeze-dried, a paracetamol medicine was obtained.

### Example 9:

Cranberry extract: hydroxypropyl methylcellulose = 25:1. 500 mg cranberry extract was injected into a 0.8 ml mould. 20 mg hydroxypropyl methylcellulose was dissolved in 1 ml water, and injected into the 500 mg powder. After stirred and freeze-dried, a cranberry solid beverage was obtained.

### Example 10:

Selegiline: polyglutamic acid = 1:10. 1 mg selegiline and 10 mg polyglutamic acid were dispersed into 0.1 ml water, and subsequently injected into a 0.1 ml mould. After frozen and freeze-dried, selegiline medicine was obtained.

### Example 11:

Vitamin B6: hydroxypropyl starch = 2:1. 10 mg vitamin B6 and 5 mg hydroxypropyl starch were dispersed into 0.1 ml water, and subsequently injected into a 0.1 ml mould. After frozen and freeze-dried, vitamin B6 medicine was obtained.

### Example 12:

Aspirin: xanthan gum + pullulan = 10:1. 500 mg aspirin, 10 mg xanthan gum and 40 mg pullulan, wherein 500 mg aspirin was injected into a 0.5 ml mould, 10 mg xanthan gum and 40 mg pullulan was dissolved in 0.5 ml water, and then injected into the 500 mg aspirin powder. After stirred, the mixture was freeze-dried to obtain aspirin medicine.

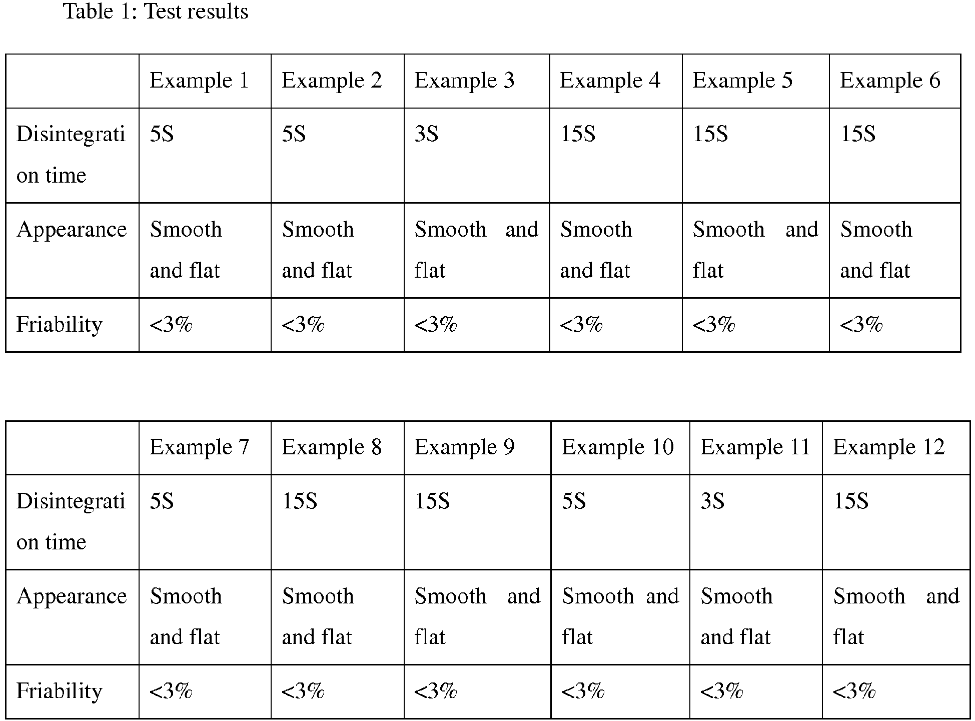

Freeze-dried forming preparation and preparation method thereof provided herein have been described by Examples, and it is apparent that modification or proper change and combination can be made to the freeze-dried forming preparation and preparation method thereof described herein by those skilled in the art, without departing from the content, spirit and scope of the invention, in order to achieve the techniques disclosed in the present invention. In particular, it should be pointed out that all similar substitutions and modifications become apparent to those skilled in the art, and they are deemed to be within the spirit, scope and content of the present invention.

## Claims

1. A freeze-dried forming preparation, which is **characterized in that**, the freeze-dried forming preparation is only composed of an active ingredient and a binder, wherein the ratio by weight between the binder and the active ingredient is in the range from 1:100 to 100:1.

2. The freeze-dried forming preparation of claim 1, which is **characterized in that**, the active ingredient is selected from the combination of one or more of chemical medicine ingredient, traditional Chinese medicine ingredient, natural extract, bioactive ingredient, and beneficial ingredient for skin care.

3. The freeze-dried forming preparation of claim 1, which is **characterized in that**, the binder is selected from gums, cellulose ethers, modified starches, PVP, carbomer, PVA, hyaluronic acids, albumin, chitosan, dextran, agar, polyamino acids, polysaccharides, or a combination thereof.

4. The freeze-dried forming preparation of claim 3, which is **characterized in that**, the gum-derived binders are selected from collagen, gelatin, hydrolyzed gelatin, acacia, xanthan gum, carrageenin, pectin, konjac glucomannan, carrageenin, locust bean gum, wood gum, locust bean gum; the cellulose ether-derived binders are selected from carboxymethyl cellulose, carboxyethyl cellulose, hydroxyethyl methylcellulose, hydroxypropyl methylcellulose *etc.*; the modified starch-derived binders are selected from pullulan, hydroxypropyl starch, hydroxylpropylmethyl starch, pregelatinized starch, amylose, carboxymethyl starch, hydroxyethyl starch, hydroxypropyl starch *etc.*; the polyamino acids are selected from polyglutamic acid, polyalanine, polylysine *etc.*; and the polysaccharides are selected from fucoidin, and inulin *etc.*

5. The freeze-dried forming preparation of claim 1, which is **characterized in that**, the freeze-dried forming preparation also contains other adjuvant, which is selected from one or more of the antioxidant, the flavoring agent and essence, the transdermal absorption enhancer, and the pH adjusting agent.

6. The freeze-dried forming preparation of claim 5, which is **characterized in that**, the antioxidants is selected from one or more of vitamin C, cyanidin, resveratrol and polyatomic phenol compounds of plant origin; the flavoring agents and essences are selected from one or more of mint taste, chocolate taste, vanilla taste, coffee taste, tea taste, maize taste, lemon taste, and milk taste *etc.*; the transdermal absorption enhancers are selected from one or more of lecithin, tween and span; the pH adjusting agents are selected from one or more of citric acid, tartaric acid, sodium bicarbonate and sodium carbonate.

7. The preparation method of the freeze-dried forming preparation of claim 1, which is **characterized in that**, the method comprises:
(a) injection molding is performed to the solution or suspensoid composed of an active ingredient, water and a binder; or injection molding is performed to a solid active ingredient and/or a binder, followed by adding water to form a suspensoid or a suspension;
(b) the solution, suspensoid or suspension obtained from step (a) is degassed in a quantitative mould;
(c) the solution, suspensoid or suspension from step (a) is directly frozen or the degassed solution, suspensoid or suspension from step (b) is frozen at low temperature;
(d) the formulation from step (c) is freeze-dried in a quantitative mould to obtain a freeze-dried forming preparation.

8. The preparation method of the freeze-dried forming preparation of claim 5, which is **characterized in that**, the method comprises:
(a) injection molding is performed to the solution or suspensoid composed of an active ingredient, water, a binder and other adjuvant; or injection molding is performed to a solid active ingredient and/or a binder and/or other adjuvant, followed by adding water to form a suspensoid or a suspension;
(b) the solution, suspensoid or suspension obtained from step (a) is degassed in a quantitative mould;
(c) the solution, suspensoid or suspension from step (a) is directly frozen or the degassed solution, suspensoid or suspension from step (b) is frozen at low temperature;
(d) the formulation from step (c) is freeze-dried in a quantitative mould to remove the solvent and obtain a freeze-dried forming preparation.
